# EUROPÄISCHE PATENTSCHRIFT

(11) **EP 0 904 767 B1**
(45) Veröffentlichungstag und Bekanntmachung des Hinweises auf die Patenterteilung: **14.12.2005**
(21) Anmeldenummer: 98118366.8
(22) Anmeldetag: 29.09.1998
(51) Int. Cl.: A61K 6/083

(54) **Dentalmassen auf der Basis von ROMP-Oligomeren oder -Polymeren**
Dental materials based on oligomers or polymers obtained by ring-opening metathesis polymerization
Matériau dentaire à base d'oligomères ou polymères obtenus par polymérisation par méthathèse par ouverture de cycle

(30) Priorität: 29.09.1997 DE 19742980
(43) Veröffentlichungstag der Anmeldung: 31.03.1999
(73) Patentinhaber: 3M ESPE AG, 82229 Seefeld (DE)
(72) Erfinder: Bissinger, Peter, 86415 Mering (DE)

(56) Entgegenhaltungen:
- EP-A- 0 792 881
- EP-A- 0 796 607
- WO-A-95/07310
- DE-A- 19 608 313

## Beschreibung

Die vorliegende Erfindung betrifft Dentalmassen, die Oligomere oder Polymere enthalten, welche durch Ring-Öffnungs-Metathese-Polymerisation erhalten werden.

In polymerisierbaren Dentalmassen werden bislang vorwiegend ethylenisch ungesättigte Monomere, bevorzugt Methacrylat- und Acrylatmonomere verwendet. Besonders häufig wird dabei das von Bowen beschriebene 2,2-Bis[4,1-phenylenoxy(2-hydroxy-3,1-propandiyl)-methacrylsäureester]-propyliden (Bis-GMA) [US-A-3 066 112] eingesetzt. Mischungen dieses Methacrylats mit Triethylenglykoldimethacrylat (TEGDMA) dienen auch heute noch als Standardrezeptur für dentale plastische Direkt-Füllungswerkstoffe. Die Aushärtung derartiger Massen beruht auf einer radikalischen Polymerisationsreaktion welche durch entsprechend aktivierte radikalbildende Initiatoren gestartet wird. Problematisch hierbei ist der bei der Polymerisation auftretende nachteilige Polymerisationsschrumpf. Dieser kann beispielsweise bei der Anwendung als Füllungsmaterial zur Bildung von Verfärbungen am Kavitätenrand des Zahnes oder sogar zur Entstehung von Randspalten mit anschließendem Sekundärkariesrisiko führen.

Neben diesen radikalisch polymerisierenden Systemen werden als dentale Füllungs- und Befestigungsmaterialien auch Zweikomponenetensysteme verwendet, die durch eine ZementReaktion zwischen einem reaktiven Füllstoff und einer mit diesem Füllstoff reagierenden Flüssigkeit bestehen. Beispiele hierfür sind die Phosphat-, Silikat-, Carboxylat- [DE-B-1 617 688] und Glasionomerzemente [DE-A-2 101889]. Eine allgemeine Übersicht über dieses Gebiet findet sich z.B. in A.D. Wilson; Chemical Society Reviews (1978), 7, (2), 265-296 oder in D. Welker, A. Rzanny, R. Göbel; Dental Magazin (1997), 2, 64-76. Diese Zementmaterialien haben gegenüber den radikalisch polymerisierenden Dentalmassen den großen Nachteil, daß ihre mechanischen Eigenschaften wie Druck- und Biegefestigkeiten deutlich schlechter sind. Die Ursache hierfür ist im Falle der Glasionomerzemente unter anderem eine recht hohe Flexibilität der Polycarbonsäure der Flüssigkeit.

Aufgabe der vorliegenden Erfindung ist es, Dentalmaterialien bereit zu stellen, die Oligomere oder Polymere enthalten welche im Falle von Zementsystemen zu besseren mechanischen Werten führen.

Gelöst wird die Aufgabe durch die Bereitstellung von Massen, welche enthalten:
(a) 10 bis 60 Gew.-%, bezogen auf (a) + (b) + (d), Monomere, Oligomere und/oder Polymere,
(b) 40 bis 85 Gew.-%, bezogen auf (a) + (b) + (d), Füllstoffe,
(c) 0,05 bis 2,0 Gew.-%, bezogen auf (a), mindestens eines Initiators oder eines Initiatorsystems, und
(d) 0 bis 30 Gew.-%, insbesondere 5 bis 30 Gew.-%, bezogen auf (a) + (b) + (d), übliche Hilfsstoffe, einschließlich Pigmente, röntgenopake Zusatzstoffe und/oder Thixotropie-Hilfsmittel,
dadurch gekennzeichnet, daß 5 bis 100 Gew.-% der Komponente (a) Oligomere- bzw. Polymere der allgemeinen Formel 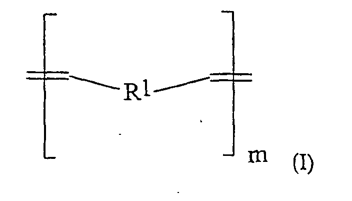 bzw. 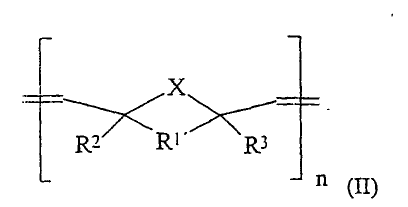 sind, in welchen bedeuten:
X gleich CH₂, NH, O oder S,
m einen Wert von 10 bis 20.000,
n einen Wert von 10 bis 20.000,
R¹ gleich -CHR⁴-CHR⁵-, 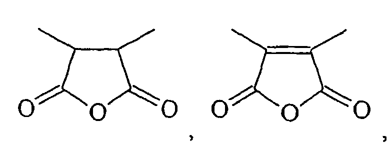 C₂-C₁₀-Alkylen, oder C₆-C₁₅-o-Arylen welches durch Alkyl, OH, NH₂, C(O)OR⁶, C(O)NHR⁶, PO₃H, SO₃H, Cl, Br oder F substituiert sein kann,
R1' gleich -CHR⁴-CHR⁵-, 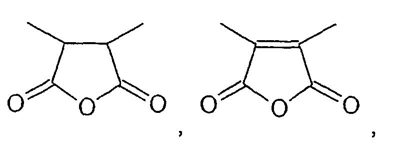 R², R³, R⁴, R⁵ gleich H, C₁-C₁₅-Alkyl, C(O)OR⁶, C(O)NHR⁶, PO₃H, SO₃H, OH und R⁶ gleich H oder einen linearen, verzweigten oder cyclischen C₁-C₃₀-Alkyl- oder -Arylrest, der 0-10 O- oder N-Atome und 0 bis 5 Carbonylgruppen enthalten kann und gesättigt oder ungesättigt ist, mit der Maßgabe, daß diese Verbindungen Gruppen enthalten, welche durch eine Zementreaktion zu einer Aushärtung der Massen führen.

Oligomere oder Polymere der allgemeinen Formeln I und II können durch Ring-Öffnungs-Metathese-Polymerisation (ROMP) erhalten werden. Die Ring-Öffnungs-Metathese-Polymerisation ist literaturbekannt und wird seit einigen Jahren auch industriell eingesetzt [Comprehensive Polymer Sci.; 4, 109-142]. Verwendungen dieser durch ROMP gewonnenen Oligomeren bzw. Polymeren für dentale Anwendungen sind nicht bekannt.

Es wurde im Rahmen dieser Erfindung überraschenderweise festgestellt, daß Verbindungen der allgemeinen Formeln (I) und (II) für dentale Zwecke gut geeignet sind und zu Massen mit besonderen Eigenschaften führen. Bei Zementsystemen erhält man bessere mechanische Werte. Dies gilt sowohl wenn die Verbindungen der Formeln (I) und (II) alleine als auch wenn sie als Mischung in den Dentalmassen enthalten sind.

Verbindung (III) mit m = 1000-3000 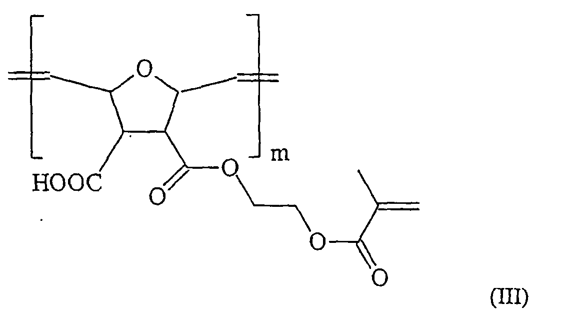 ist ein ethylenisch ungesättigtes Oligomeres bzw. Polymeres welches in den offenbarten Dentalmassen entweder alleine oder in Kombination mit weiteren ethylenisch ungesättigten Monomeren eingesetzt werden kann. Bevorzugt verwendete ethylenisch ungesättigte Co-Monomere sind Acrylate oder Methacrylate. Besonders bevorzugte ethylenisch ungesättigte Co-Monomere sind Bis-GMA, TEGDMA, Bis-(hydroxymethyl)tricyclo[5.2.1.0^{2,6}]-decandiacrlysäureester sowie 2,2-Bis[4,1-phenylenoxy(3,1-propandiyl)-methacrylsäureester]-propyliden.

Weitere bevorzugte Verbindungen sind den Formeln (IV) - (VIII) zu entnehmen (mit m = 1000-3000):

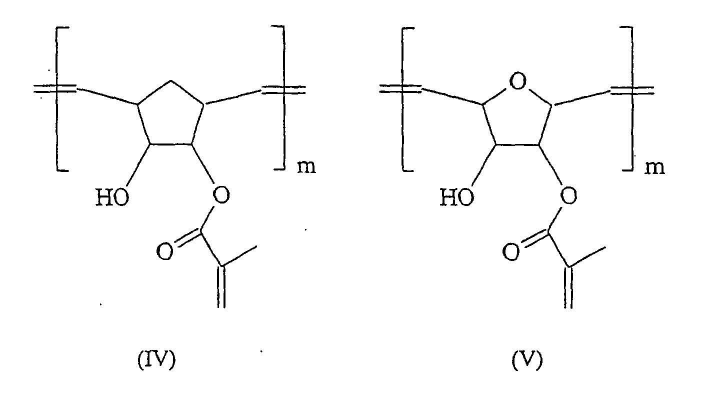

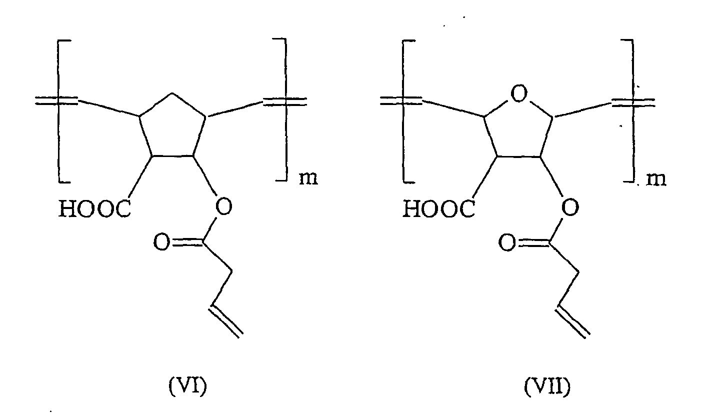

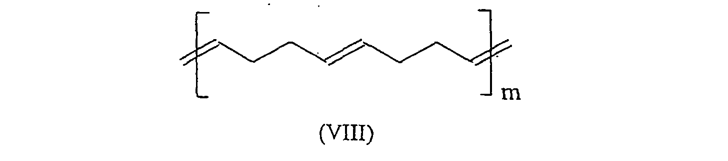

Die Synthese der Verbindungen (III)-(VII) erfolgt dabei jeweils durch eine ROMP entsprechender Norbonenderivate, welche wiederum durch Diels-Alder-Reaktion gewonnen werden. Eine allgemeine Beschreibung dieser Reaktionsfolgen ist z.B. in "Comprehensive organometallic Chemistry II: a review of the literature 1982-1994", Elsevier 1995, Seiten 1209-1232 zu finden. Die Verbindung (VIII) kann gemäß den Angaben aus A. Demonceau et al. in Macromolecules (1997), 30 Seiten 3127 bis 3136 hergestellt werden.

Radiakalbildende Katalysatoren gemäß Bestandteil (c) zur Aushärtung dieser ethylenisch ungesättigten Monomeren, Oligomeren und Polymeren können durch UV- oder sichtbares Licht aktivierbare Substanzen sein, wie z.B. Benzoinalkylether, Benzilketale, Acylphosphinoxide oder aliphatische und aromatische 1,2-Diketonverbindungen, z.B. Campherchinon, wobei die Lichtpolymerisation durch Zusatz von Aktivatoren, wie tertiären Aminen oder organischen Phosphiten, in an sich bekannter Weise beschleunigt werden kann.

Geeignete Initiatorsysteme zur Auslösung der radikalischen Polymerisation über einen Redox-Mechanismus sind beispielsweise die Systeme Peroxid/Amin oder Peroxid/Barbitursäurederivate u. dergl. Bei Verwendung solcher Initiatorsysteme ist es zweckmäßig, einen Initiator (z.B. Peroxid) und eine Katalysatorkomponente (z.B. Amin) getrennt bereitzuhalten. Die beiden Komponenten werden dann kurz vor ihrer Anwendung miteinander homogen vermischt.

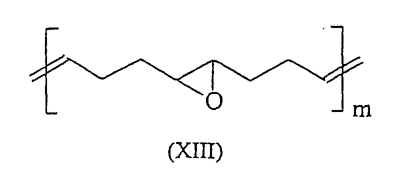

Zur Aushärtung von epoxyfunktionalisierten Monomeren, Oligomeren bzw. Polymeren werden gemäß Bestandteil (c) Kationenbildner eingesetzt. Als Kationenbildner verwendet werden können Säurebildner wie z.B. Lewis oder Broensted-Säuren bzw. Verbindungen, die solche Säuren freisetzen, welche die kationische Polymerisation initiieren, beispielsweise BF₃ oder dessen etherische Addukte (BF₃*THF, BF₃*Et₂O, etc.), AlCl₃, FeCl₃, HPF₆, HAsF₆, HSbF₆, HBF₄ oder Substanzen, die nach Bestrahlen durch UV oder sichtbares Licht oder durch Wärme und/oder Druck die Polymerisation auslösen, wie z.B. (eta-6-Cumol)(eta-5-cyclopentadienyl)eisen-hexafluorophosphat, (eta-6-Cumol)(eta-5-cyclopentadienyl)eisen-tetrafluoroborat, (eta-6-Cumol)(eta-5-cyclopentadienyl) eisen-hexafluoroantimonat, substituierte Diaryliodoniumsalze und Triarylsulfonium-Salze, verwendet. Als Beschleuniger können Peroxyverbindungen vom Typ der Perester, der Diacylperoxide, der Peroxydicarbonate und der Hydroperoxide eingesetzt werden. Bevorzugt werden Hydroperoxide verwendet, als besonders bevorzugter Beschleuniger kommt Cumolhydroperoxid in etwa 70-90 %iger Lösung in Cumol zum Einsatz. Das Verhältnis vom Photoinitiator zum Cumolhydroperoxid kann in weiten Grenzen von 1 : 0,001 bis 1 : 10 variiert werden, vorzugsweise wird jedoch ein Verhältnis von 1 : 0,1 bis 1 : 6 und besonders bevorzugt von 1 : 0,5 bis 1 : 4 verwendet. Die Verwendung von Komplexbildnern, wie beispielsweise Oxalsäure, 8-Hydroxychinolin, Ethylendiamintetraessigsäure und aromatischen Polyhydroxyverbindungen ist ebenfalls möglich. Als Verzögerer können Basen, typischerweise tertiäre Amine, zugesetzt werden.

Geeignete Füllstoffe, welche in Verbindung mit den ethylenisch ungesättigten bzw. epoxyfunktionalisierten Oligomeren oder Polymeren eingesetzt werden können, sind in der Regel anorganische Füllstoffe. Beispielhaft genannt seien Quarz, gemahlene Gläser, Kieselgele sowie pyrogene Kieselsäuren oder deren Granulate. Bevorzugt werden röntgenopake Füllstoffe, zumindest teilweise, mit eingesetzt. Diese können zum einen röntgenopake Gläser sein, also Gläser, welche z.B. Strontium, Barium oder Lanthan enthalten, oder ein Teil der Füllkörper besteht aus einem röntgenopaken Zusatz, wie beispielsweise Yttriumtrifluorid, Strontiumhexafluorozirkonat oder Fluoriden der Selten-Erdmetalle. Zum besseren Einbau in die Polymermatrix ist es von Vorteil, die anorganischen Füllstoffe zu hydrophobieren. Übliche Hydrophobierungsmittel sind Silane, beispielsweise Trimethoxymethacryloyloxypropylsilan, oder Trimethoxyglycidylsilan. Die Füllkörper haben vorzugsweise eine mittlere Kornverteilung <20 µm und insbesondere <5 µm sowie eine obere Korngrenze von 150, vorzugsweise 70 µm und insbesondere 25 µm. Besonders bevorzugt werden Gemische von 5-25 Gew.-% Füllstoffe mit einer mittleren Korngröße von 0,02 - 0,06 µm und 65-85 Gew.-% Füllkörper mit einer mittleren Korngröße von 1-5 µm verwendet.

Verbindungen der allgemeinen Formeln (I) und (II), welche durch eine Zementreaktion abbinden, enthalten vorzugsweise freie Carboxylgruppen. Bislang werden z.B für die sog. Glasionomerzemente Polycarbonsäuren verwendet, denen als Monomere Acrylsäure, Methacrylsäure, Maleinsäure, Fumarsäure oder Itaconsäure zu Grunde liegen. Nachteilig an Zementen die mit solchen Polycarbonsäuren abbinden ist, daß sie im Vergleich zu anderen dentalen Füllungsmaterialien nur sehr schlechte Biegefestigkeitswerte aufweisen. Eine Ursache hierfür wird in der hohen Flexibiltät der Polycarbonsäureketten gesehen, die zu keiner genügenden Steiffigkeit des ausgehärteten Materials führen. Versuche die Carboxyl-Gruppendichte pro Monomereinheit zu steigern führen meist zu Polymeren, welche für die Anwendung in einem Zementsystem nicht mehr genügend wasserlöslich sind.

Durch ROMP lassen sich aus 5,6-Carboxyl-substituierten Norbornenen literaturbekannte Polymersäuren herstellen, die in ihrer Polymerkette gemäß den Formeln (I) und (II) eine C-C-Doppelbindung und einen gesättigten Fünfring aufweisen. Diese Polymersäuren weisen ein Molgewicht pro Carbonylgruppe von 105-107 g/Mol auf und sind überraschenderweise trotz ihrer deutlich niedrigeren Carboxyl-Gruppendichte, verglichen mit herkömmlichen Polymersäuren, sehr gut wasserlöslich. Darüber hinaus binden diese durch ROMP gewonnenen Polymersäuren mit säurelöslichen Glaspulvern unter Zementbildung ab. Zudem bieten diese Säuren die 9 Möglichkeit durch Folgereaktionen weiter modifiziert zu werden. So kann beispielsweise die Kettenlänge dieser Moleküle durch oxidative Spaltung gemäß dem folgenden Schema dem jeweiligen Anwendungsbedürfnis angepasst werden.

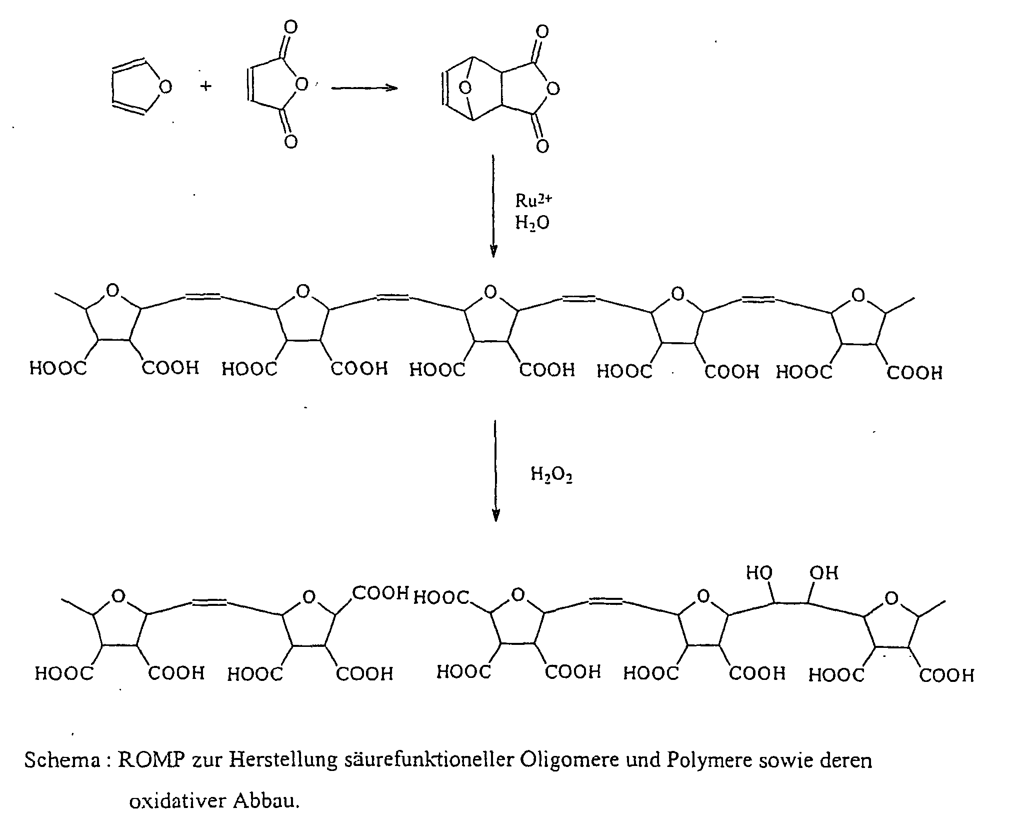

Besonders bevorzugte Verbindungen die in Zementen eingesetzt werden können sind in den Formeln (XIV) bis (XXIV) dargestellt (mit m=1000 bis 3000):

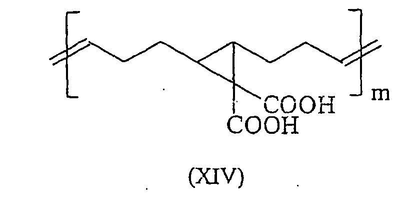

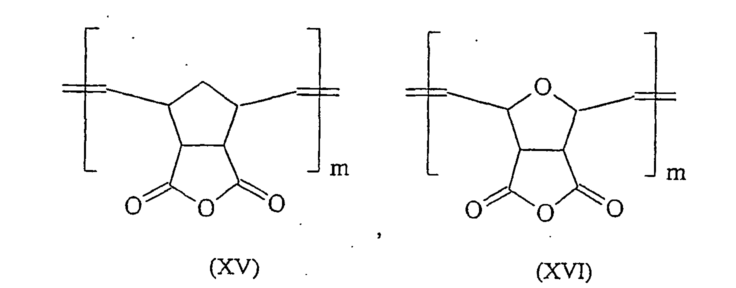

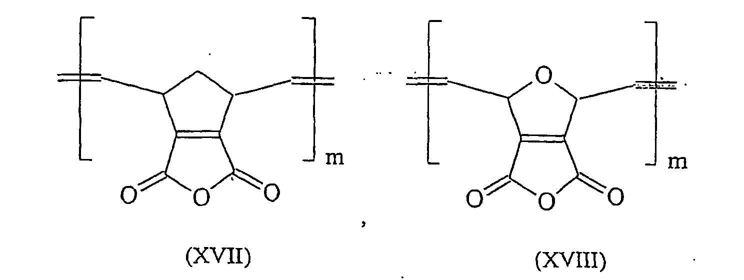

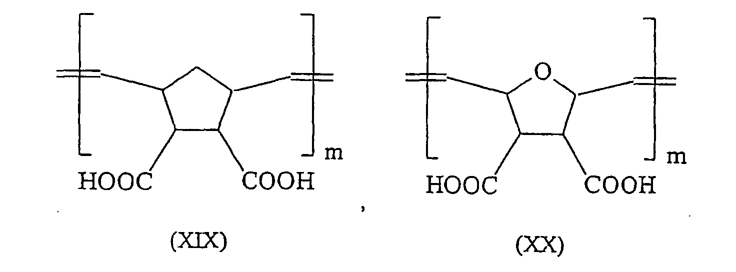

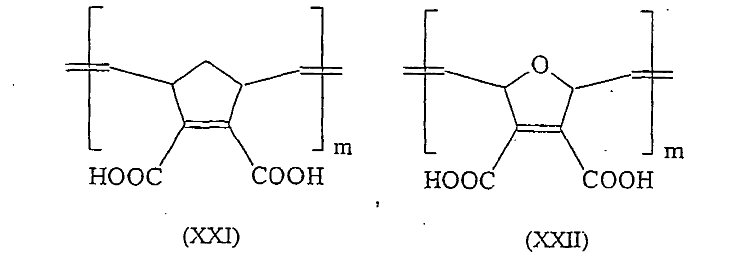

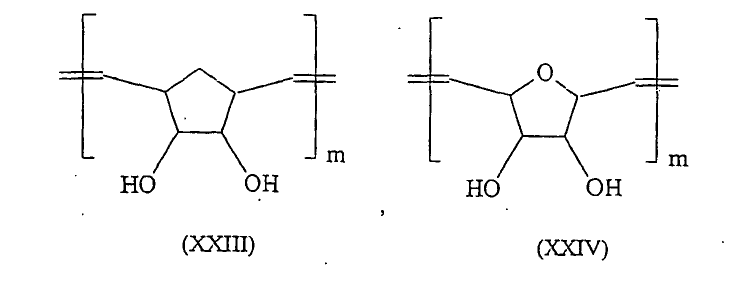

Die Verbindung (XIV) kann gemäß den Angaben aus A. Demonceau et al. in Macromolecules (1997), 30 Seiten 3127 bis 3136 hergestellt werden. Die Synthese der Verbindungen (XV) bis (XXIV) kann dabei wieder aus den entsprechenden Vorstufen wie in "Comprehensive organometallic Chemistry II: a review of the literature 1982-1994", Elsevier 1995, Seiten 1209-1232 beschrieben durchgeführt werden.

Zur Verwendung in Dentalmassen können die hier beschriebenen Polymersäuren entweder alleine oder in Kombination mit bekannten und für Zemente bereits eingesetzten Säuren eingebracht werden. Solche Säuren sind beispielhaft in der DE-A-2 101 889 beschrieben.

Als säurelösliche Pulver gemäß Bestandteil (b) können herkömmliche Silikatzementpulver verwendet werden, wie sie ebenfalls in der DE-A-2 101 889 beschrieben sind. Besonders vorteilhaft ist jedoch die Verwendung von Calcium-aluminium-fluoro-silikatglaspulvem, die in der DE-A-2 061 513 beschrieben sind. Geeignete Silikatzementpulver sind ausserdem in "Chemical Society Reviews" (1978), 7 (2), 265-296 angegeben.

Die Dentalzemente werden gewöhnlich in Form eines Zweikomponentensystems angewendet, welches aus einer flüssigen und einer pulverförmigen Komponente besteht. Die erfindungsgemäßen Oligomer- bzw. Polymersäuren können gemäß einer Ausführungsform i) der Erfindung Teil der Anrührflüssigkeit und gemäß einer anderen Ausführungsform ii) Teil der Pulverkomponente sein:

In dem zuerst genannten Fall (i) besteht die Anrührflüssigkeit aus einer wässrigen Lösung der vorstehend definierten Oligomer- bzw. Polymer-Säuren sowie von herkömmlichen Polymersäuren und gegebenenfalls üblichen Zusätzen. Die säurehaltigen Monomere liegen im allgemeinen in einer Konzentration von mindestens 20 %, meist 30 bis 60 Gew.-%, insbesondere 40 bis 50 Gew.-% in der wässrigen Lösung vor, wenn das Zementsystem als Zahnfüllzement bestimmt ist. Für Befestigungszemente und prothetische Zwecke sind bereits niedrigere Konzentrationen vorteilhaft. Die wässrigen Lösungen sollten Viskositäten von mindestens 0,5 Poise, jedoch höchstens 300 Poise haben. Ein bevorzugter Viskositätsbereich liegt zwischen 2 und 200, insbesondere zwischen 5 und 100 Poise (bei 25°C).

Es ist üblich, Dentalzemente prädosiert in sogenannten Schüttelkapseln zu vertreiben. In diesen werden Flüssigkeit und Pulver in zwei getrennten Kammern vorgesehen und unmittelbar vor Gebrauch vereinigt und mechanisch gemischt. Diese Prädosierung ist auch auf die erfindungsgemäßen Dentalmassen anwendbar.

Gemäß der weiteren vorteilhaften Ausführungsform (ii) der Erfindung ist die Anmischkomponente der Dentalzemente, d. h. die Oligomer- bzw. Polymer-Säuren gemäß Bestandteil (a), Bestandteil des Pulvergemisches und liegt als Vormischung aus Glaspulver und säurefunktionellen Oligomeren bzw. Polymeren vor. Die Anmischkomponente kann dann einfach mit Wasser, gegebenenfalls mit den übliche Zusätzen angemischt werden.

Zwischen beiden Ausführungsformen i) und ii) sind fließende Übergänge möglich: So können z.B. die Oligomer- und Polymer-Säuren je zur Hälfte der flüssigen und der pulverförmigen Anmischkomponente zugesetzt werden.

Auch bei der Ausführungsform ii) kann es zweckmäßig sein, sie prädosiert in Schüttelkapseln vorzusehen und zu vertreiben. Es kann auch besonders vorteilhaft sein, das Pulvergemisch zu einer Tablette zu verpressen.

Neben der Anwendung in Dentalzementen können die Verbindungen XIV bis XXIV auch als Adhäsivvermittler in sogenannten Bondingzusammensetzungen verwendet werden. Diese sind entweder ungefüllt (0 Gew.-% an Komponente (b)) oder niedrig gefüllt (5 bis 30 Gew.-% an Komponente (b)).

Geeignete Hilfsstoffe gemäß Komponente (d) können beispielsweise üblicherweise auf dem Dentalgebiet eingesetzte Stabilisatoren, Pigmente oder Verdünnungsmittel sein. Im Falle der zementabbindenden Systeme können sowohl der Pulver- als auch der flüssigen Komponente Chelatbildner zugesetzt werden, vorzugsweise Weinsäure, um die Aushärtecharakteristik zu verbessern [s. DE-A-2 319 715].

Die erfindungsgemäßen Dentalmassen eigenen sich zur Herstellung von Füllungsmaterialien, Befestigungszementen, Inlays, Onlays, Verblendschalen, provisorischen Kronen- und Brückenmaterialien, zahntechnischen Werkstoffen sowie Abformmaterialien.

Im Folgenden soll die Erfindung durch Beispiele näher erläutert werden.

### Beispiele

### Beispiel 1: Synthese und oxidativer Abbau von Poly-7-oxabicyclo-[2.2.1]-hept-5-en-2,3-dicarbonsäure

30 g Exo-7-oxabicyclo[2.2.1]-hept-5-en-2,3-dicarbonsäureanhydrid werden zusammen mit einer Lösung aus K₂[RuCl₅]*xH₂O in 105 g Wasser auf 60°C unter Rühren erwärmt. Nach etwa 45 Minuten entsteht eine klare hochviskose Lösung. Diese wird auf 90°C erwärmt und innerhalb von einer Stunde tropfenweise mit insgesamt 10,0 g 30 %-iger Wasserstoffperoxidlösung versetzt. Anschließend wird die Lösung bis zur Trockne eingeengt und mit Wasser auf etwa 50 % verdünnt.

### Beispiel 2: Herstellung eines Glasionomerzements mit erfindungsgemäßen Polymersäuren

200 mg der wässrigen Lösung aus Beispiel 1 werden mit 200 mg einer wässrigen Polycarbonsäurelösung (CHELON-FIL-Flüssigkeit, Fa. ESPE, Seefeld) vermischt. Diese Mischung wird mit 1,2 g reaktivem Glaspulver (CHELON-FIL-Pulver, Fa. ESPE, Seefeld) zu einer Paste angespatelt. Zur Bestimmung der Druck- und Biegefestigkeitswerte wird der frisch angemischte Zement in die entsprechenden Prüfkörperformen eingebracht und verbleibt dort bis zum Abbindeende (ca. 5 Minuten). Anschließend werden die Prüfkörper entformt und an einer Zwick-Universal-Prüfmaschine entsprechend der Norm ISO-Norm 4049 bzw. der ISO-Norm 9917 vermessen. Die Ergebnisse der Materialprüfung sind Tabelle 1 zu entnehmen.

### Referenzbeispiel 1: Herstellung eines einkomponentigen radikalisch polymerisierenden Dental-Füllungs-Materials

30g Exo-7-oxabicyclo[2.2.1]-hept-5-en-2,3-dicarbonsäureanhydrid werden in 23,5 g Hydroxethylenmethacrylat (HEMA) gelöst und 10 Stunden gerührt. Anschließend wird mit einer Lösung aus 105 g Wasser und K₂[RuCl₅]*xH₂O (c= 140mg/ml) aufgenommen und unter Rühren auf 60°C erwärmt. Nach etwa 45 Minuten entsteht eine klare hochviskose Lösung, welche für mehre Stunden am Hochvakuum getrocknet wird. Anschließend wird der zähfließende Rückstand mit 10 g Triethylenglykoldimethacrylat (TEGDMA) verdünnt. 10 g dieser Mischung werden mit 10 g Bis-(hydroxymethyl)-tricyclo[5.2.1.0^{2,6}]-decandiacrylsäureester und 0,07 g Campherchinon gemischt und anschließend mit 0,5 g hochdispersem Siliciumdioxid (Aerosil OX50, Fa. Degussa) sowie 79,5 g feingemahlenem Quarzpulver zu einer homogenen Paste verknetet. Zur Bestimmung der Druck-und Biegefestigkeitswerte wird die Paste in die entsprechenden Prüfkörperformen eingebracht und entsprechend den Angaben der ISO-Norm 4049 bzw. der ISO-Norm 9917 belichtet. Die Bestimmung des Volumenschrumpfes erfolgt durch Linometermessung. Die Ergebnisse der Materialprüfung sind Tabelle 2 zu entnehmen.

### Referenzbeispiel 2: Herstellung eines einkomponentigen kationisch polymerisierenden Dental-Füllungs-Materials

10 g des Polymers welches aus Monomer 9 des Literaturzitates A. Demonceau, A.W. Stumpf, E. Saive, A.F. Noels; Macromolecules (1997), 30 3127-3136 durch ROMP erhältlich ist, wird mit 10 g 3,4-Epoxycyclohexylmethyl-3',4'-epoxycyclohexylcarboxylat, 0,8.g Ferroceniumhexafluorantimonat und 0,9 g Cumolhydroperoxid gemischt und anschließend mit 0,5 g hochdispersem Siliciumdioxid (Aerosil OX50, Fa. Degussa) sowie 79,5 g feingemahlenem Quarzpulver zu einer homogenen Paste verknetet. Zur Bestimmung der Druck- und Biegefestigkeitswerte wird die Paste in die entsprechenden Prüf-körperformen eingebracht und entsprechend den Angaben der ISO-Norm 4049 bzw. der ISO-Norm 9917 belichtet. Die Bestimmung des Volumenschrumpfes erfolgt durch Linometermessung. Die Ergebnisse der Materialprüfung sind Tabelle 3 zu entnehmen.

**Tabelle 1**

| Mechanische Eigenschaften des erfindungsgemäßen Glasionomerzementes im Vergleich zu einem bekannten Glasionomerzement | | |
|---|---|---|
| | Beispiel 2 | Ketac-Fil (Fa. ESPE, Seefeld) |
| Druckfestigkeit [MPa]^{a)} | 157 | 165 |
| Biegefestigkeit [MPa]^{b)} | 52 | 35 |

| | | |
|---|---|---|
| a) Gemessen gemäß ISO-Norm 4049 | | |
| b) Gemessen gemäß ISO-Norm 9917 | | |

**Tabelle 2**

| Mechanische Eigenschaften der Dentalmasse gemäß Referenzbeispiel 1 im Vergleich zu einem bekannnten Komposit-Füllungsmaterial | | |
|---|---|---|
| | Beispiel 3 | Pertac II (Fa. ESPE, Seefeld) |
| Druckfestigkeit [Mpa]^{a)} | 412 | 420 |
| Biegefestigkeit [MPa]^{a)} | 98 | 110 |
| Volumenschrumpf [%]^{c)} | 1,6 | 2,3 |

| | | |
|---|---|---|
| a) Gemessen gemäß ISO-Norm 4049 | | |
| c) Gemessen mit ACTA-Linometer (A.J. de Gee, A.J. Feilzer, C.L. Davidson; Dent Mat (1993), 9, 11-14) | | |

**Tabelle 3**

| Mechanische Eigenschaften der Dentalmasse gemäß Referenzbeispiel 2 im Vergleich zu einem bekannnten Komposit-Füllungsmaterial | | |
|---|---|---|
| | Beispiel 4 | Pertac II (Fa. ESPE, Seefeld) |
| Druckfestigkeit [Mpa]^{a)} | 390 | 420 |
| Biegefestigkeit [MPa]^{a)} | 90 | 110 |
| Volumenschrumpf [%]^{c)} | 1,4 | 2,3 |

| | | |
|---|---|---|
| a) Gemessen gemäß ISO-Norm 4049 b) Gemessen gemäß ISO-Norm 9917 | | |
| c) Gemessen mit ACTA-Linometer (A.J. de Gee, A.J. Feilzer, C.L. Davidson; Dent Mat (1993), 9, 11-14) | | |

## Patentansprüche

1. Dentalzement enthaltend
(a) 10 bis 60 Gew.-%, bezogen auf (a) + (b) + (d), Oligomere und/oder Polymere,
(b) 40 bis 85 Gew.-%, bezogen auf (a) + (b) + (d), Füllstoffe,
(c) 0,05 bis 2,0 Gew.-%, bezogen auf (a), mindestens eines Initiators oder eines Initiatorsystems,
(d) 0 bis 30 Gew.-%, bezogen auf (a) + (b) + (d), übliche Hilfsstoffe, einschließlich Pigmente, röntgenopake Zusatzstoffe und/oder Thixotropie-Hilfsmittel,
**dadurch gekennzeichnet, daß** 5 bis 100 Gew.-% der Komponente (a) Oligomere- bzw. Polymere der allgemeinen Formel 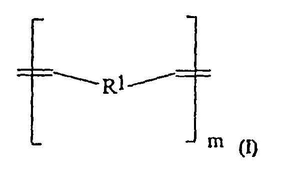 bzw. 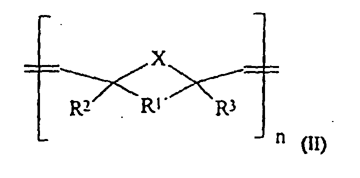 sind, in welchen bedeuten:
X gleich CH₂, NH, O oder S,
m einen Wert von 10 bis 20.000,
n einen Wert von 10 bis 20.000,
R¹ gleich -CHR⁴-CHR⁵-, 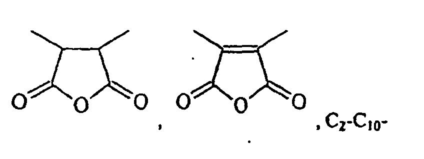 Alkylen oder C₆-C₁₅-o-Arylen welches durch Alkyl, OH, NH₂, C(O)OR⁶, C(O)NHR⁶, PO₃H, SO₃H, Cl, Br oder F substituiert sein kann,
R1' gleich -CHR⁴-CHR⁵-. 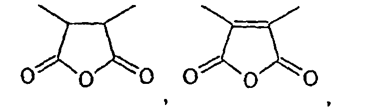 R², R³, R⁴, R⁵ gleich H, C₁-C₁₅-Alkyl, C(O)OR⁶, C(O)NHR⁶, PO₃H, SO₃H, OH und
R⁶ gleich H oder einen linearen, verzweigten oder cyclischen C₁-C₃₀-Alkyl- oder-Arylrest, der 0-10 O- oder N-Atome und 0 bis 5 Carbonylgruppen enthalten kann und gesättigt ist, mit der Maßgabe, daß diese Verbindungen Gruppen enthalten, welche durch eine Zementreaktion zu einer Aushärtung der Massen führen.

2. Dentalzement nach Anspruch 1, **dadurch gekennzeichnet, daß** er aus einer Pulverkomponente und einer Anmischflüssigkeit besteht und der Bestandteil (a) in der Pulverkomponente enthalten ist.

3. Dentalzement nach Anspruch 1, **dadurch gekennzeichnet, daß** er aus einer Pulverkomponente und einer Anmischflüssigkeit besteht und der Bestandteil (a) in der Anmischflüssigkeit enthalten ist.

4. Verwendung des Dentalzements nach den Ansprüchen 1 bis 3 zur Herstellung von Füllungsmaterialien, Befestigungszementen, Inlays, Onlays, Verblendschalen, provisorischen Kronen- und Brückenmaterialien, zahntechnischen Werkstoffen sowie Modellmaterialien.

## Claims

1. Dental cement, comprising
(a) 10 to 60% by weight, based on (a) + (b) + (d), of oligomers and/or polymers,
(b) 40 to 85% by weight, based on (a) + (b) + (d), of fillers,
(c) 0.05 to 2.0% by weight, based on (a), of at least one initiator or one initiator system,
(d) 0 to 30% by weight, based on (a) + (b) + (d), of conventional auxiliaries, including pigments, additives opaque to X-rays and/or thixotropic auxiliaries,
**characterized in that** 5 to 100% by weight of the component (a) are oligomers or polymers of the general formula 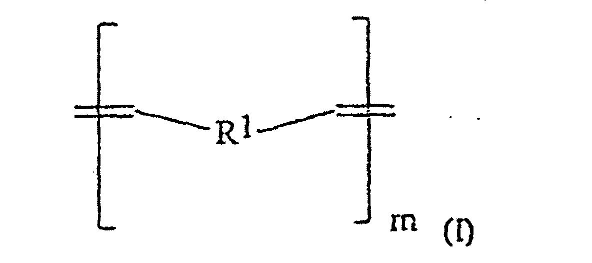 or 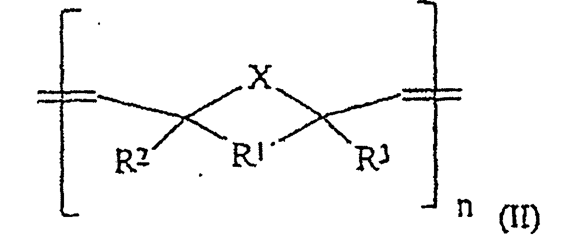 in which:
X equally well represents CH₂, NH, O or S,
m represents a value of 10 to 20 000,
n represents a value of 10 to 20 000,
R¹ equally well represents -CHR⁴-CHR⁵-, 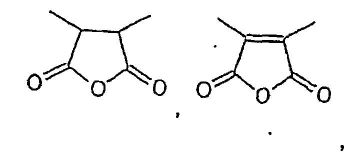 C₂-C₁₀-alkylene or C₆-C₁₅-o-arylene, which can be substituted by alkyl, OH, NH₂, C(O)OR⁶, C(O)NHR⁶, PO₃H, SO₃H, Cl, Br or F,
R1' equally well represents -CHR⁴-CHR⁵-, 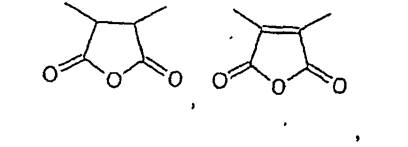 R², R³, R⁴ and R⁵ equally well represent H, C₁-C₁₅-alkyl, C(O)OR⁶, C(O)NHR ₆ PO₃H, SO₃H or OH, and
R⁶ equally well represents H or a linear, branched or cyclic C₁-C₃₀-alkyl or -aryl radical which can comprise 0-10 oxygen or nitrogen atoms and 0 to 5 carbonyl groups and is saturated,
with the proviso that these compounds comprise groups which result, by a cement reaction, in setting of the materials.

2. Dental cement according to Claim 1, **characterized in that** it consists of a powder component and of a mixing liquid and the constituent (a) is comprised in the powder component.

3. Dental cement according to Claim 1, **characterized in that** it consists of a powder component and of a mixing liquid and the constituent (a) is comprised in the mixing liquid.

4. Use of the dental cement according to Claims 1 to 3 in the preparation of filling materials, fixing cements, inlays, onlays, veneers, temporary crown and bridge materials, materials for dental technology and model materials.

## Revendications

1. Ciment dentaire, contenant
(a) 10 à 60% en poids, par rapport à (a) + (b) + (d), d'oligomères et/ou de polymères,
(b) 40 à 85% en poids, par rapport à (a) + (b) + (d), de charges,
(c) 0,05 à 2,0% en poids, par rapport à (a), d'au moins un initiateur ou d'un système d'initiateurs,
(d) 0 à 30% en poids, par rapport à (a) + (b) + (d), d'adjuvants usuels, y compris des pigments, des additifs opaques aux rayons X et/ou des adjuvants thixotropiques,
**caractérisé en ce** 5 à 100% en poids du composant (a) sont des oligomères ou des polymères de formules générales 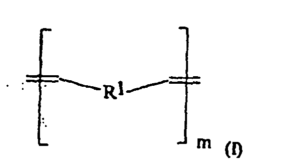 ou 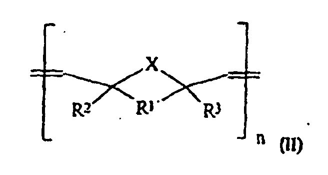 dans lesquelles :
X signifie CH₂, NH, O ou S,
m vaut 10 à 20000,
n vaut 10 à 20000,
R¹ signifie -CHR⁴-CHR⁵-, 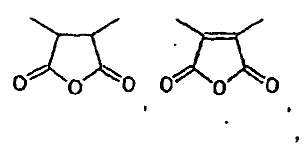 alkylène en C₂ à C₁₀ ou O-arylène en C₆ à C₁₅, qui peut être
substitué par alkyle, OH, NH₂, C(O)OR⁶, C(O)NHR⁶, PO₃H, SO₃H, Cl, Br ou F,
R¹' signifie -CHR⁴-CHR⁵-, 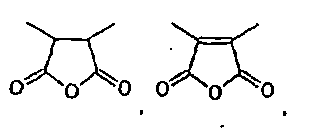 R², R³, R⁴, R⁵ signifient H, alkyle en C₁ à C₁₅, C(O)OR⁶, C(O)NHR⁶, PO₃H, SO₃H, OH et
R⁶ signifie H ou un radical alkyle ou aryle en C₁ à C₃₀ linéaire, ramifié ou cyclique, qui peut contenir 0 à 10 atomes O ou N et 0 à 5 groupes carbonyle et qui est saturé, à condition que ces composés contiennent des groupes qui conduisent à un durcissement des masses par une réaction cimentaire.

2. Ciment dentaire selon la revendication 1, **caractérisé en ce qu'**il est constitué par un composant sous forme de poudre et un liquide de gâchage et le constituant (a) est contenu dans le composant sous forme de poudre.

3. Ciment dentaire selon la revendication 1, **caractérisé en ce qu'**il est constitué par un composant sous forme de poudre et un liquide de gâchage et le constituant (a) est contenu dans le liquide de gâchage.

4. Utilisation du ciment dentaire selon les revendications 1 à 3 pour la réalisation de matériaux de remplissage, de ciments de fixation, d'inlays, d'onlays, de coquilles de recouvrement, de matériaux pour couronnes et bridges provisoires, de matériaux techniques dentaires et de matériaux de moulage.
